Europäisches Patentamt

European Patent Office

Office européen des brevets

(19)

(11) Numéro de publication: **0 231 048**
**A1**

(12) ## DEMANDE DE BREVET EUROPEEN

(21) Numéro de dépôt: 87200103.7

(22) Date de dépôt: 26.01.87

(51) Int. Cl.⁴: **G 01 S 15/52**
**G 01 S 7/52**
**// A61B8/08**

(30) Priorité: 31.01.86 FR 8601348

(43) Date de publication de la demande:
05.08.87 Bulletin 87/32

(84) Etats contractants désignés:
**BE DE ES FR GB SE**

(71) Demandeur: **Laboratoires d'Electronique et de Physique Appliquée L.E.P.**
**3, Avenue Descartes**
**F-94450 Limeil-Brévannes (FR)**

(84) Etats contractants désignés: **FR**

(71) Demandeur: **N.V. Philips' Gloeilampenfabrieken**
**Groenewoudseweg 1**
**NL-5621 BA Eindhoven (NL)**

(84) Etats contractants désignés: **BE DE ES GB SE**

(72) Inventeur: **Bernatets, Jean-Luc Société Civile S.P.I.D.**
**209, rue de l'Université**
**F-75007 Paris (FR)**

(74) Mandataire: **Landousy, Christian et al**
**Société Civile S.P.I.D. 209, Rue de l'Université**
**F-75007 Paris (FR)**

(54) **Appareil d'examen de milieux en mouvement par échographie ultrasonore.**

(57) L'appareil comprend au moins un transducteur ultrasonore (10) associé à un étage d'émission (20) et à un étage (30) de réception et de traitement des signaux échographiques renvoyés vers ledit transducteur. Ce dernier étage (30) comprend une première voie de traitement classique (60) composée essentiellement d'un circuit d'amplification (61), d'un circuit de détection d'enveloppe (62), d'un convertisseur de balayage (64), et d'un dispositif de visualisation (65), ainsi qu'une deuxième voie de traitement (100) comprenant :

(A) un convertisseur analogique-numérique (63) et une première mémoire d'image (64) pour le stockage de l'image en cours d'acquisition ;

(B) un circuit de détection de mouvement (130) commandant ou non, pour chaque point d'image, la mise à jour d'un coefficient de filtrage α ;

(C) un circuit de filtrage temporel récursif conditionnel (150) délivrant en sortie de ladite deuxième voie de traitement (100) une image traitée R(t,x,y) qui est une somme de l'image nouvellement acquise I(t,x,y) et de l'image précédemment traitée R(t-1,x,y) pondérées par des valeurs liées à celle dudit coefficient de filtrage α.

Application : Echographes médicaux

EP 0 231 048 A1

**Description**

APPAREIL D'EXAMEN DE MILIEUX EN MOUVEMENT PAR ECHOGRAPHIE ULTRASONORE

La présente invention concerne un appareil d'examen de milieux en mouvement par échographie ultrasonore comprenant au moins un transducteur ultrasonore associé à un étage d'émission répétée de signaux ultrasonores vers le milieu à explorer et à un étage de réception et de traitement des signaux échographiques renvoyés vers ledit transducteur comprenant lui-même une première voie de traitement composée essentiellement d'un circuit d'amplication, d'un circuit de détection d'enveloppe, d'un convertisseur de balayage, et d'un dispositif de visualisation.

La mise en oeuvre d'un tel examen est décrite par exemple dans l'article "Cardiac Ultrasonography" de D.L. King paru dans la revue Radiology 103, mai 1972, pages 387 à 392, dans le cas d'une application à l'imagerie cardiaque. L'échographie par ultrasons constitue en effet l'un des moyens privilégiés pour l'examen de tissus biologiques, grâce à son innocuité et à sa capacité de visualiser en temps réel les structures internes d'organes. Néanmoins, la qualité des images ultrasonores est plus faible que celle attachée aux autres méthodes d'imagerie.

Le but de l'invention est de proposer un appareil d'échographie ultrasonore dans lequel on met à profit le fait que l'acquisition des images est réalisable en temps réel pour effectuer des traitements temporels destinés à améliorer la qualité de ces images, et ce sans perte de résolution spatiale soit par dégradation des hautes fréquences spatiales de l'image, soit en raison du flou de bouger introduit par le mouvement des tissus ou des organes.

A cet effet, l'invention concerne un appareil tel que défini dans le préambule ci-dessus et caractérisé en ce que l'étage de réception comprend une deuxième voie de traitement comprenant elle-même :

(A) un convertisseur anologique-numérique et une première mémoire d'image pour le stockage de l'image en cours d'acquisition notée $I(t,x,y)$ où $t$ est le temps et $x$ et $y$ les coordonnées des points d'image ;

(B) un circuit de détection de mouvement prévu pour commander ou non, pour chaque point d'image et selon la valeur par rapport à un seuil déterminé de la différence entre un point de l'image acquise $I(t,x,y)$ et le point correspondant de l'image de sortie dite $R(t-1, x,y)$ de la deuxième voie de traitement, la mise à jour d'un coefficient de filtrage temporel récursif $\alpha$ ;

(C) un circuit de filtrage temporel récursif conditionnel prévu pour délivrer en sortie de ladite deuxième voie de traitement une image traitée $R(t,x,y)$ qui est une somme de l'image nouvellement acquise $I(t,x,y)$ et de l'image précédemment traitée $R(t-1,x,y)$ pondérées par des valeurs liées à celle dudit coefficient de filtrage $\alpha$. Le convertisseur analogique-numérique et la première mémoire d'image sont de préférence ceux du convertisseur de balayage.

La structure ainsi proposée permet, dans un premier temps, de détecter les zones de l'image où un mouvement a eu lieu et, dans un deuxième temps, de conditionner les traitements temporels à la détection de tels mouvements, lesdits traitements n'étant effectués que si l'on peut considérer qu'il n'y a pas eu de mouvement d'une image à la suivante.

Dans un mode de réalisation particulier de l'invention, le circuit de détection de mouvement comprend en série : un soustracteur pour l'évaluation de ladite différence entre $I(t,x,y)$ et $R(t-1,x,y)$ fournies respectivement par la première mémoire d'image et une deuxième mémoire d'image, une troisième mémoire d'image pour le stockage alternativement de ladite différence et du coefficient de filtrage $\alpha$, un filtre passe-bas, et un comparateur prévu pour comparer la valeur de son signal d'entrée à un seuil déterminé et pour commander selon le résultat de la comparaison le remplacement du contenu de la troisième mémoire d'image par une valeur prédéterminée constituant alors le nouveau coefficient de filtrage $\alpha$.

Dans un autre mode de réalisation, le circuit de détection de mouvement comprend en série : un soustracteur pour l'évaluation de ladite différence entre $I(t,x,y)$ et $R(t-1,x,y)$ fournies respectivement par la première mémoire d'image et une deuxième mémoire d'image, une troisième mémoire d'image pour le stockage alternativement de ladite différence et du coefficient de filtrage $\alpha$, un filtre passe-bas, et une quatrième mémoire prévue pour commander le remplacement du contenu de la troisième mémoire d'image par une valeur de substitution d'autant plus faible que le contenu stocké dans ladite quatrième mémoire est plus grand, et inversement, ladite valeur de substitution constituant alors le nouveau coefficient de filtrage $\alpha$.

Dans l'un ou l'autre de ces exemples de réalisation, le circuit de filtrage temporel récursif peut comprendre un premier et un deuxième multiplieur pour la multiplication respectivement du coefficient de filtrage $\alpha$ par la sortie de l'une des première et troisième mémoires d'image et du complément à 1 dudit coefficient (c'est-à-dire $1-\alpha$) par la sortie de l'autre desdites mémoires d'image, un additionneur des sorties desdits multiplieurs, et la deuxième mémoire d'image de stockage des images traitées par ledit circuit de filtrage temporel récursif.

Enfin, le traitement des points d'image peut être séquentiel, avec une seule deuxième voie de traitement pour l'ensemble des points, ou en parallèle, avec autant de deuxièmes voies de traitement que de points, ou peut être réalisé selon un compromis de ces deux solutions, avec plusieurs deuxièmes voies de traitement en parallèle traitant chacune séquentiellement une fraction correspondante des points.

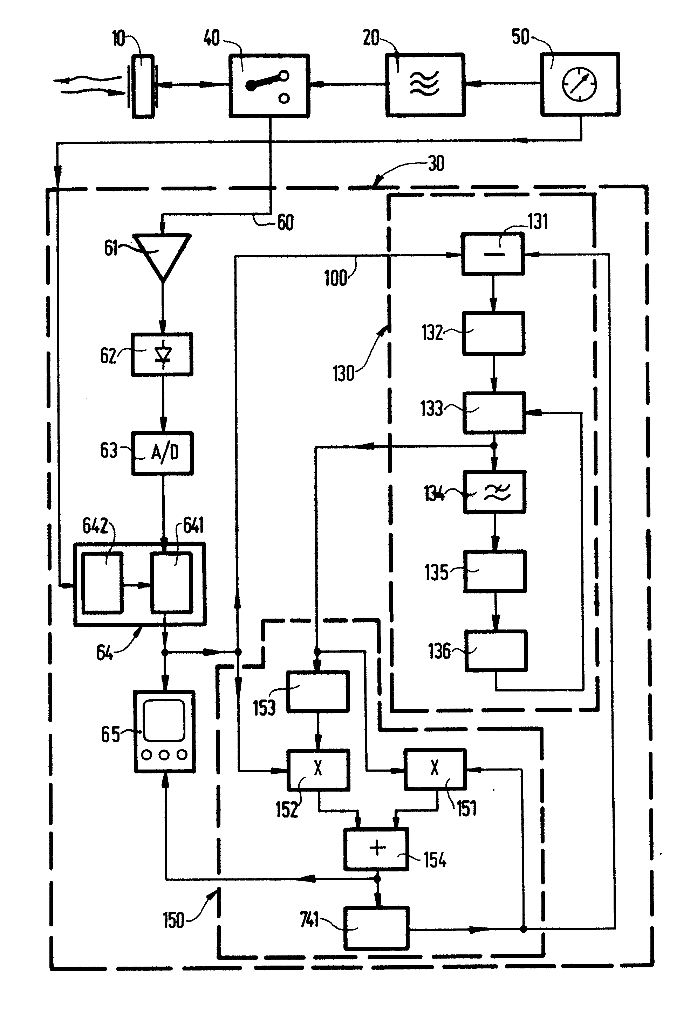

Les particularités et avantages de l'invention apparaîtront maintenant de façon plus précise dans la description qui suit et sur la figure annexée, donnée à titre d'exemple et qui montre un mode de réalisation de l'appareil selon l'invention.

L'appareil comprend, dans ce mode de réalisation, un transducteur ultrasonore 10 auquel sont associés d'une part un étage 20 d'émission répétée de signaux ultrasonores vers le milieu à explorer et d'autre part un étage 30 de réception et de traitement des signaux échographiques renvoyés vers ledit transducteur. Un circuit d'aiguillage 40 peut être inséré entre les étages d'émission et de réception, pour éviter notamment l'aveuglement de ce dernier par l'étage d'émission, ou, de façon équivalente, un circuit de protection peut être prévu aux mêmes fins en tête de l'étage de réception et de traitement.

Un circuit d'horloge 50 assure de façon classique le séquencement nécessaire, en imposant d'une part le rythme de répétition des images, à une fréquence de l'ordre de 8 à 40 hertz par exemple, et d'autre part le rythme de répétition des lignes (environ 128 lignes par image). Pour chaque nouvelle image, le transducteur 10 est replacé dans sa position initiale puis balaye à nouveau tout le champ d'exploration ligne après ligne. Pour chaque ligne, une première voie de traitement 60 reçoit les signaux électriques délivrés par le transducteur 10 en correspondance aux signaux échographiques renvoyés vers lui. Ces signaux sont amplifiés dans un amplificateur 61 puis redressés et filtrés dans un détecteur d'enveloppe 62 avant d'être représentés sur un dispositif de visualisation 65.

Pour permettre cette représentation, les enveloppes sont numérisées dans un convertisseur analogique-numérique 63 puis envoyées dans un convertisseur de balayage 64 (en anglais: scan-converter). Ce convertisseur 64 contient en fait une mémoire d'image 641, où sont écrites les valeurs des points de l'image. Un circuit d'écriture/lecture 642 assure d'une part cette écriture et d'autre part la relecture pour l'affichage de l'image enregistrée.

Conformément à l'invention, l'étage de réception de l'appareil comprend une deuxième voie de traitement 100, elle-même composée successivement d'un circuit de détection de mouvement 130 par comparaison d'images successives et d'un circuit de filtrage temporel récursif conditionnel 150.

Le circuit de détection de mouvement 130 comprend lui-même, tout d'abord, un soustracteur 131, qui calcule pour chaque point d'image la différence entre un point de l'image qui vient d'être acquise par le convertisseur d'image 64 et le point correspondant de l'image précédente qui vient d'être visualisée et qui a été stockée dans une deuxième mémoire d'image temporaire 741. Cette différence est alors envoyée vers une troisième mémoire d'image 133, en sortie de laquelle est prévu un filtre passe-bas 134. Le filtrage ainsi effectué est destiné à éliminer les points isolés dont la contribution provient du bruit aléatoire, qu'on veut justement éliminer. Il peut en particulier, être un filtrage spatial obtenu par exemple en remplaçant chaque point d'image par une moyenne arithmétique de points d'image calculée dans une fenêtre n x n centrée sur ce point d'image (on a choisi ici n = 3). La notion de filtrage spatial d'une image et des méthodes de mise en oeuvre d'un tel filtrage sont déjà bien connues et ne seront pas davantage décrites ici.

La sortie du filtre passe-bas 134 est envoyée vers un comparateur 136 qui applique un seuil point à point à l'image filtrée. Si, en un point, le signal filtré est en valeur absolue inférieur ou égal au seuil, on décide qu'il n'y a pas eu mouvement au voisinage de ce point et qu'un filtrage inter-images va pouvoir être effectué. Si au contraire le signal filtré est en valeur absolue supérieur audit seuil, on décide qu'il y a eu mouvement et que l'on va renoncer à réaliser ce filtrage temporel inter-images. Selon le résultat de la comparaison ainsi effectuée, les valeurs stockées dans la troisième mémoire d'images 133 sont mises à jour par substitution de la valeur "zéro" (mouvement) ou d'un coefficient prédéterminé (absence de mouvement) à celle primitivement contenue dans cette mémoire 133 (et qui avait servi à calculer la valeur reçue par le comparateur).

La sortie de la troisième mémoire 133, appelée maintenant coefficient de filtrage $\alpha$, est envoyée vers le circuit de filtrage temporel récursif conditionnel 150. Ledit filtrage est effectué, on l'a vu, entre une image traitée au temps (t-1) et une image acquise au temps t, sans préciser pour le moment la nature de l'intervalle de temps (t-1, t). Pour abréger la désignation "point d'image", on emploiera l'expression pixel (de l'anglais "picture element") dans la suite de la description, chaque pixel étant donc repéré par sa position (x,y) au sein de chaque image. En appelant I(t,x,y) l'image initialement acquise, dépendante de x, de y et du temps t, R(t,x,y,) l'image résultante après le filtrage temporel récursif, et $\alpha$ un coefficient dit d'efficacité du filtrage et compris entre 0 et 1, on décide que ledit filtrage sera effectué conformément à l'expression suivante :

$$R(t,x,y) = \alpha(t,x,y) \cdot R(t-1,x,y) + (1- \alpha(t,x,y)) \cdot I(t,x,y)$$

On constate, à la lecture de cette expression, qu'$\alpha$ détermine réellement l'efficacité du filtrage, puisque :

- si $\alpha$ est faible, on filtre peu les images, le terme $((1-\alpha).I)$ étant prépondérant (et si $\alpha$ est très faible, on ne filtre quasiment pas les images, ce qui équivaut à un filtrage inopérant) ;

- si $\alpha$ est fort, c'est au contraire le terme $(\alpha .R)$ qui est prépondérant, mais si $\alpha$ est trop fort, cette prépondérance devient telle que les informations contenues dans la dernière image acquise I(t,x,y) sont quasiment négligées et que seule compte l'influence de la première image traitée, ce qui équivaut une nouvelle fois à un traitement de filtrage inopérant.

En pratique, pour que le filtrage soit correct, il convient donc de choisir $\alpha$ de façon appropriée. Les essais réalisés ont montré que $(1- \alpha)$ devait être à peu près égal à l'inverse du nombre d'images nécessaire pour que le traitement soit perçu, et qu'une valeur de $\alpha$ égale à 7/8 semblait pour cela être un bon choix.

Le circuit de filtrage temporel récursif conditionnel 150, qui reçoit donc la sortie de la troisième mémoire d'image 133, comprend un premier multiplieur 151, recevant ladite sortie de la troisième mémoire 133, et un deuxième multiplieur 152, recevant grâce à la présence d'une quatrième mémoire 153 le complément à 1 du coefficient $\alpha$

calculé pour chaque pixel par le circuit de détection de mouvement 130. Cette quatrième mémoire 153 contient pour chaque valeur dudit coefficient $\alpha$, utilisée comme adresse, la valeur du complément à 1 cherchée. Le multiplieur 151 effectue ici la multiplication de la sortie de la deuxième mémoire d'image 741 (c'est-à-dire l'image précédemment traitée et visualisée après le traitement lui donnant la forme R(t-1)) par la sortie de la troisième mémoire 133, et le multiplieur 152 effectue la multiplication de la sortie de la première mémoire d'image 641 du convertisseur de balayage 64 (c'est-à-dire la nouvelle image acquise I(t)) par le complément à 1 (issu de la quatrième mémoire 153) de la même sortie de la troisième mémoire 133. On pourrait également effectuer les multiplications symétriques, c'est-à-dire celle de la sortie de la mémoire 641 par la sortie de la mémoire 133 et celle de la sortie de la mémoire 741 par le complément à 1 de la sortie de la mémoire 133.

Le circuit 150 comprend également un additionneur 154 recevant les sorties des deux multiplieurs 151 et 152 et délivrant un signal égal à l'expression déjà citée :

$$R(t) = \alpha.R(t-1) + (1- \alpha).I(t)$$

La sortie de cet additionneur 154 est envoyée d'une part vers le dispositif de visualisation 65 et d'autre part vers la deuxième mémoire d'image 741 qui assure le stockage temporaire de l'image ainsi reçue, appelée par la suite image précédente R(t-1), lorsque arrivera la nouvelle image acquise I(t). Ce stockage temporaire de l'image traitée est nécessaire jusqu'à la réalisation du traitement de l'image suivante, la nouvelle image qui va être acquise.

Avec les notations adoptées précédemment, on voit donc que le circuit de détection de mouvement 130, décrit en détail plus haut et qui précède le circuit de filtrage temporel récursif conditionnel 150, opère dans le soustracteur 131 la différence entre les signaux I(t), nouvelle image acquise, et R(t-1), image précédemment traitée par filtrage conditionnel et visualisée. Cette opération est effectuée en vue d'opérations ultérieures de filtrage conditionnel dans le circuit 150 conduisant à la détermination d'une nouvelle image traitée R(t) qui sera à son tour soustraite de I(t+1) dans le soustracteur 131, et ainsi de suite.

Bien entendu, la présente invention n'est pas limitée à l'exemple de réalisation décrit et représenté, à partir duquel des variantes peuvent être proposées sans pour cela sortir du cadre de l'invention.

En particulier, il est manifeste que l'appareil selon l'invention peut comprendre non plus un seul transducteur, mais une barrette de transducteurs, linéaire ou bidimensionnelle, éventuellement associée à un dispositif de balayage électronique.

Par ailleurs, lors de la détection de mouvement effectuée par le circuit 130, on peut remplacer la différence entre images successives par une grandeur fonction de cette différence, par exemple par son carré, ou par une fonction plus complexe. Ladite grandeur est, dans tous les cas, calculée alors par un premier circuit de calcul 132 intercalé en sortie du soustracteur 131 et composé en général d'une mémoire morte délivrant la valeur de ladite grandeur correspondant à celle de la différence en sortie dudit soustracteur. De même, en sortie du filtre passe-bas 134, un deuxième circuit de calcul 135 peut être prévu pour remplacer la valeur de sortie dudit filtre par une fonction liée à cette valeur, par exemple à la valeur absolue de cette sortie du filtre. Si l'un ou l'autre des circuits de calcul 132 ou 135, ou les deux, sont prévus, c'est bien entendu la sortie du circuit 132 qui est envoyée vers la troisième mémoire d'image 133, et la sortie du circuit 135 qui est fournie au comparateur 136.

Dans une variante de réalisation, le comparateur 136 peut être remplacé par un circuit de calcul du type mémoire morte. Ce circuit de calcul peut alors soit commander exactement les mêmes opérations de substitution que celles prévues ci-dessus selon le résultat des comparaisons, soit fournir une valeur de substitution à placer dans la troisième mémoire d'image 133. Ladite valeur est d'autant plus faible que le contenu de cette mémoire est grand, et inversement. Dans ce dernier cas, au lieu d'effectuer une comparaison par tout ou rien, on réalise un filtrage temporel inter-images d'autant plus faible que la probabilité d'existence de mouvement au point d'image considéré est plus forte, et inversement. Ce filtrage progressif ou dégressif peut être réalisé par exemple en ne limitant plus la valeur de $\alpha$, le coefficient d'efficacité de filtrage, à un choix binaire (7/8 s'il n'y a pas détection de mouvement, 0 s'il y a détection de mouvement) mais en faisant varier $\alpha$ progressivement avec la plus ou moins grande présomption de mouvement.

On doit également préciser que plusieurs modes de réalisation sont possibles pour les moyens d'exécution de toutes les opérations effectuées point à point. En effet ces opérations peuvent être réalisées en séquence, point après point, par un seul circuit. Ces opérations peuvent au contraire être réalisées en parallèle, par autant de circuits qu'il y a de points dans l'image (par exemple 256 x 256), cette solution accélérant grandement le traitement mais augmentant bien entendu la complexité des circuits de l'appareil. Ces opérations peuvent enfin être réalisées en choisissant un compromis entre les deux solutions précédentes, à l'aide de plusieurs circuits (en nombre plus faible que le nombre de points de l'image) qui sont placés en parallèle pour traiter séquentiellement chacun une partie des points.

Enfin on notera bien que, dans le cas de l'échocardiographie ou de l'examen de tout organe à mouvement cyclique, un appareil mettant en oeuvre l'invention telle que décrite nécessiterait de conserver en mémoire toutes les images d'un cycle complet, c'est-à-dire une seconde d'images environ dans le cas d'un cycle cardiaque. Comme un échographe de bonne qualité dans le domaine cardiaque fournit environ 50 images par seconde, le stockage correspondant pourrait être problématique. Deux solutions sont principalement envisageables : adjoindre à l'échographe soit un disque numérique dit "temps réel", de haute capacité et à grande vitesse d'accès, soit un nombre suffisant de mémoires de trame. Un tel disque ou de telles mémoires de trame sont actuellement disponibles industriellement sans problème.

Une troisième solution peut être proposée, qui consiste à réduire la taille mémoire nécessaire. On peut en effet demander à l'appareil selon l'invention de moindres performances et, au lieu de lui faire traiter toutes les images du cycle cardiaque, limiter ses possibilités au traitement d'un nombre restreint d'images, par exemple au traitement d'instants privilégiés repérés sur l'échocardiogramme (fin de systole, fin de diastole, accidents nettement repérables sur l'échocardiogramme, etc...). Mais pour déterminer de tels instants privilégiés, il est nécessaire de se synchroniser sur le cycle à observer. Une telle synchronisation est par exemple décrite, pour d'autre types d'examen cardiaque, dans le brevet US-A-4547892, et ne sera donc pas davantage détaillée ici. Les mouvements du coeur ou ceux du thorax étant à peu près périodiques, cette synchronisation fait en sorte que les différentes parties de l'organe considéré se retrouvent à peu près à la même place dans chaque cycle. Cette caractéristique permet de réaliser le filtrage temporel selon l'invention sans que la détection de mouvement soit contrainte d'en annuler les effets.

**Revendications**

1. Appareil d'examen de milieux en mouvement par échographie ultrasonore comprenant au moins un transducteur ultrasonore associé à un étage d'émission répétée de signaux ultrasonores vers le milieux à explorer et à un étage de réception et de traitement des signaux échographiques renvoyés vers ledit transducteur comprenant lui-même une première voie de traitement composée essentiellement d'un circuit d'amplification, d'un circuit de détection d'enveloppe, d'un convertisseur de balayage, et d'un dispositif de visualisation, caractérisé en ce que l'étage de réception comprend une deuxième voie de traitement comprenant elle-même :

(A) un convertisseur analogique-numérique et une première mémoire d'image pour le stockage de l'image en cours d'acquisition, notée $I(t,x,y)$ où $t$ est le temps et $x$ et $y$ les coordonnées des points d'image ;

(B) un circuit de détection de mouvement prévu pour commander ou non, pour chaque point d'image et selon la valeur par rapport à un seuil déterminé de la différence entre un point de l'image acquise $I(t,x,y)$ et le point correspondant de l'image de sortie dite $R(t-1,x,y)$ de la deuxième voie de traitement, la mise à jour d'un coefficient de filtrage temporel récursif $\alpha$ ;

(C) un circuit de filtrage temporel récursif conditionnel prévu pour délivrer en sortie de ladite deuxième voie de traitement une image traitée $R(t,x,y)$ qui est une somme de l'image nouvellement acquise $I(t,x,y)$ et de l'image précédemment traitée $R(t-1,x,y)$ pondérées par des valeurs liées à celle dudit coefficient de filtrage $\alpha$.

2. Appareil selon la revendication 1, caractérisé en ce que le convertisseur analogique-numérique et la première mémoire d'image de la deuxième voie de traitement sont ceux du convertisseur de balayage.

3. Appareil selon l'une des revendications 1 et 2, caractérisé en ce que le circuit de détection de mouvement comprend en série : un soustracteur pour l'évaluation de ladite différence entre $I(t,x,y)$ et $R(t-1,x,y)$ fournies respectivement par la première mémoire d'image et une deuxième mémoire d'image, une troisième mémoire d'image pour le stockage alternativement de ladite différence et du coefficient de filtrage $\alpha$, un filtre passe-bas, et un comparateur prévu pour comparer la valeur de son signal d'entrée à un seuil déterminé et pour commander selon le résultat de la comparaison le remplacement du contenu de la troisième mémoire d'image par une valeur prédéterminée constituant alors le nouveau coefficient de filtrage $\alpha$.

4. Appareil selon l'une des revendications 1 et 2, caractérisé en ce que le circuit de détection de mouvement comprend en série : un soustracteur pour l'évaluation de ladite différence entre $I(t,x,y)$ et $R(t-1,x,y)$ fournies respectivement par la première mémoire d'image et une deuxième mémoire d'image, une troisième mémoire d'image pour le stockage alternativement de ladite différence et du coefficient de filtrage $\alpha$, un filtre passe-bas, et une quatrième mémoire prévue pour commander le remplacement du contenu de la troisième mémoire d'image par une valeur de substitution d'autant plus faible que le contenu stocké dans ladite quatrième mémoire est plus grand, et inversement, ladite valeur de substitution constituant alors le nouveau coefficient de filtrage $\alpha$.

5. Appareil selon l'une des revendications 3 et 4, caractérisé en ce que le circuit de filtrage temporel récursif comprend un premier et un deuxième multiplieur pour la multiplication respectivement du coefficient de filtrage $\alpha$ par la sortie de l'une des première et troisième mémoires d'image et du complément à 1 dudit coefficient, c'est-à-dire $1 - \alpha$, par la sortie de l'autre desdites mémoires d'image, un additionneur des sorties desdits multiplieurs, et la deuxième mémoire d'image de stockage des images traitées par ledit circuit de filtrage temporel récursif.

6. Appareil selon l'une des revendications 3 à 5, caractérisé en ce que la deuxième voie de traitement comprend en série, en sortie du soustracteur, un pemier circuit de calcul pour l'évaluation d'une grandeur fonction du signal de sortie dudit soustracteur et constituant le coefficient de filtrage $\alpha$ à stocker dans la troisième mémoire d'image.

7. Appareil selon l'une des revendications 3 à 6, caractérisé en ce que la deuxième voie de traitement comprend en série, en sortie du filtre passe-bas, un deuxième circuit de calcul pour l'évaluation d'une grandeur fonction du signal de sortie dudit filtre et constituant la nouvelle valeur de signal à comparer au seuil déterminé.

8. Appareil selon l'une des revendications 6 et 7, caractérisé en ce que le premier et le deuxième circuit de calcul sont des mémoires mortes.

9. Appareil selon l'une des revendications 1 à 8, caractérisé en ce qu'il comprend en parallèle plusieurs deuxièmes voies de traitement pour traiter séquentiellement une partie des points d'image seulement.

10. Appareil selon l'une des revendications 1 à 8, caractérisé en ce qu'il comprend en parallèle autant de deuxièmes voies de traitement qu'il y a de points d'image pour traiter en parallèle tous ces points d'image.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | IEEE TRANSACTIONS ON SONICS AND ULTRASONICS, vol. SU-24, no. 6, novembre 1977, pages 350-354, New York, US; R.W. BARNES et al.: "An ultrasound digital moving target indicator system for diagnostic use" <br> * Page 350: résumé; partie II: premier alinéa; page 51, alinéa 3 - page 352, fin partie II * | 1,3,5, 6,8 | G 01 S  15/52 <br> G 01 S  7/52 // <br> A 61 B  8/08 |
| Y | FR-A-2 368 189 (MICRO CONSULTANTS LTD) <br> * Page 7, ligne 22 - page 8, ligne 22; page 13, ligne 30 - page 14, ligne 34; page 20, ligne 12 - page 21, ligne 7; page 23, ligne 13 - page 37, ligne 9 * | 1,3,5, 6,8 | |
| A | EP-A-0 025 730 (ETAT FRANCAIS-CNET) <br> * Résumé; page 5, ligne 9 - page 9, ligne 2 * | 1,3-6 | **DOMAINES TECHNIQUES RECHERCHES (Int. Cl.4)** <br><br> G 01 S <br> G 06 F <br> H 04 N <br> A 61 B |
| A | EP-A-0 113 269 (THOMSON-CSF) <br> * Résumé; page 17, ligne 25 - page 30, ligne 5 * | 1 | |
| A | WO-A-8 502 080 (INDEPENDENT BROADCASTING AUTHORITY) <br> * Résumé; page 5, ligne 30 - page 7, dernière ligne * | 1 | |

--- -/-

Le présent rapport de recherche a été établi pour toutes les revendications

| Lieu de la recherche | Date d'achèvement de la recherche | Examinateur |
|---|---|---|
| LA HAYE | 20-03-1987 | OLDROYD D.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen
des brevets

**RAPPORT DE RECHERCHE EUROPEENNE**

Numéro de la demande

EP    87 20 0103

| | **DOCUMENTS CONSIDERES COMME PERTINENTS** | | | Page    2 |
|---|---|---|---|---|
| Catégorie | Citation du document avec indication, en cas de besoin, des parties pertinentes | | Revendication concernée | CLASSEMENT DE LA DEMANDE (Int. Cl.4) |
| A | GB-A-2 138 237   (ROBERT BOSCH) <br> * En entier * | | 1 | |
| A | FR-A-2 421 523   (NIPPON ELECTRIC) <br> * En entier * | | 1 | |
| A | FR-A-2 396 313   (RAYTHEON) | | | |
| A | IEEE 1981 ULTRASONICS SYMPOSIUM PROCEEDINGS, Chicago, IL, 14-16 octobre 1981, vol. 2, pages 669-672, IEEE, New York, US; S.I. KIM et al.: "A recursive scheme for improvement of the lateral resolution in B-scan ultrasonography" | | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 20-03-1987 | OLDROYD D.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82